Europäisches Patentamt

European Patent Office

Office européen des brevets

(11) Publication number: **0 404 417**
**A1**

## EUROPEAN PATENT APPLICATION

(21) Application number: 90306348.5

(22) Date of filing: 11.06.90

(51) Int. Cl.⁵: **C07C 29/74, C07C 31/12**

(30) Priority: 19.06.89 US 367581

(43) Date of publication of application:
27.12.90 Bulletin 90/52

(84) Designated Contracting States:
BE DE FR GB NL

(71) Applicant: **TEXACO CHEMICAL COMPANY**
**3040 Post Oak Boulevard**
**Houston, Texas 77056(US)**

(72) Inventor: **Keating, Kenneth Patrick**
**24 Oakwood Drive**
**Georgetown, Texas 78626(US)**
Inventor: **Walton, John Monte**
**Route 3, Box 990**
**Georgetown, Texas 78626(US)**
Inventor: **Sanderson, John Ronald**
**P.O. Box 587**
**Leander, Texas 78641(US)**

(74) Representative: **Brock, Peter William et al**
**URQUHART-DYKES & LORD 91 Wimpole**
**Street**
**London W1M 8AH(GB)**

(54) **Removal of acidic impurities from tertiary butyl hydroperoxide containing feedstock.**

(57) Oxidation of isobutane (14) by reaction with oxygen (12) in a reaction zone (10), forms a crude mixture (16) comprising unreacted isobutane, tertiary butyl hydroperoxide, tertiary butyl alcohol and carboxylic acid impurities, including acetic acid and formic acid. The acid impurities are removed by contacting the crude mixture (16) with water (82) in zone (80), forming an extract fraction (86) comprising water, tertiary butyl alcohol and said carboxylic acid impurities, and a raffinate (84) comprising isobutane, tertiary butyl hydroperoxide and tertiary butyl alcohol.

The raffinate (84) can be separated in a distillation zone (90) into a tertiary butyl hydroperoxide/tertiary butyl alcohol fraction (24), and an unreacted isobutane distillate fraction (15), which can be combined with the isobutane (14) and recycled to a reaction zone (10).

EP 0 404 417 A1

# REMOVAL OF ACIDIC IMPURITIES FROM TERTIARY BUTYL HYDROPEROXIDE CONTAINING FEEDSTOCK

This invention relates to the purification of a tertiary butyl hydroperoxide-containing feedstock. More particularly, this invention relates to a method for the preparation of an isobutane-tertiary butyl alcohol-tertiary butyl hydroperoxide feedstock useful in the preparation of tertiary butyl alcohol, propylene oxide, etc. Still more particularly, this invention relates to a method wherein an initial reaction product obtained by the oxidation of isobutane with oxygen and comprising unreacted isobutane, tertiary butyl alcohol, tertiary butyl hydroperoxide and a minor amount of water soluble acidic by-products, including acetic acid, formic acid, etc., is extracted with water to provide a final feedstock comprising tertiary butyl alcohol, tertiary butyl hydroperoxide, ditertiary butyl peroxide, and unreacted isobutane which is substantially free of water soluble acidic impurities.

It is known to react oxygen with isobutane to form a peroxidation reaction product wherein the principal peroxide that is formed is tertiary butyl hydroperoxide. However, minor amounts of water-soluble and hydrocarbon-soluble impurities such as ditertiary butyl peroxide, acetic acid, formic acid, methyl formate, acetone, methanol, etc., are formed during the oxygenation reaction. These impurities, especially the carboxylic acids, adversely effect the quality of the tertiary butyl alcohol product. The carboxylic acids such as formic acid and acetic acid are corrosive to equipment and detrimental to subsequent operations.

A minor amount of other peroxides, including ditertiary butyl peroxide are also formed. Generally speaking, from about 10 to about 100 parts of tertiary butyl hydroperoxide are formed per part of ditertiary butyl peroxide.

The production of propylene oxide together with a coproduct, such as tertiary butyl alcohol, is described in Kollar U. S. Patents No. 3,350,422 and 3,351,635 which are directed to the catalytic epoxidation of an olefin by reaction with a hydroperoxide such as tertiary butyl hydroperoxide in solution in a solvent such as tertiary butyl alcohol. When the olefin is propylene and the hydroperoxide is tertiary butyl hydroperoxide, the principal reaction products are propylene oxide and additional tertiary butyl alcohol.

Grane U. S. Patent No. 3,474,151 is also directed to the oxidation of isobutane with oxygen to provide tertiary butyl alcohol and discloses that the reaction mixture contains not only tertiary butyl hydroperoxide, but also ditertiary butyl peroxide. In their U. S. Patent No. 4,239,926, Grane et al. disclose a method for significantly purifying the tertiary butyl alcohol prepared by the oxidation of isobutane, the method involving extractive distillation of the tertiary butyl alcohol product using a specially proportioned blend of xylene, acetone and water as the extractant.

The product of the reaction of tertiary butyl hydroperoxide with propylene is normally separated into useful components, usually by distillation, to form, for example, sequential distillate fractions composed of unreacted propylene, propylene oxide and tertiary butyl alcohol.

The contaminating impurities are normally soluble to at least a limited extent in tertiary butyl alcohol and are usually removed during the final stages of the process. For example, Grane et al. in U. S. Patents No. 3,474,151 and No. 3,239,926 and Worrell et al. in U. S. Patent No. 4,296,263 use a combination of thermal treating steps and fractionation steps in purifying the tertiary butyl alcohol.

Sanderson et al. utilize a combination of catalytic hydrogenation and distillation in purifying the tertiary butyl alcohol. See U. S. Patent No. 4,704,482 dated November 3, 1987, U. S. Patent No. 4,742,179 dated May 3, 1988 and U. S. Patent No. 4,705,903 dated November 10, 1987.

Harvey U. S. Patent No. 3,449,217 is directed to a method for the recovery of tertiary butyl hydroperoxide from a mixture of tertiary butyl hydroperoxide and tertiary butyl alcohol.

Tertiary butyl hydroperoxide is useful as a raw material for the manufacture of tertiary butyl alcohol either by the decomposition of the tertiary butyl hydroperoxide as such, or by the catalytic reaction of tertiary butyl hydroperoxide with an olefin. When the tertiary butyl hydroperoxide is reacted with propylene the principle reaction products are tertiary butyl alcohol and propylene oxide. Carboxylic acids such as formic acid and acetic acid are detrimental in thse operations even when present in small amounts.

In accordance with the present invention, after isobutane has been thermally and/or catalytically reacted with molecular oxygen to provide a crude initial feedstock composed of unreacted isobutane, tertiary butyl hydroperoxide, tertiary butyl alcohol and oxygenated impurities including carboxylic acids such as formic acid and acetic acid, the crude initial feedstock is treated with water (preferably in a counter-current extraction zone) in order to preferentially remove a significant portion of the carboxylic acid impurities and to provide a final feedstock from which unreacted isobutane may be removed (e.g., by distillation) for recycle. The resultant mixture composed of tertiary butyl hydroperoxide dissolved in tertiary butyl alcohol may be further processed for thermal and/or catalytic conversion of the tertiary butyl hydroperoxide to tertiary butyl alcohol in the manner disclosed, for example, in Grane et al. U. S. Patents No. 3,474,151 and No. 4,239,926 and Worrell et al. U. S. Patent No. 4,296,263.

In the alternative, the solution of tertiary butyl hydroperoxide in tertiary butyl alcohol may be used as a charge stock for the coproduction of tertiary butyl alcohol and propylene oxide as disclosed for example, in Kollar U. S. Patents No. 3,350,422 and No. 3,351,635.

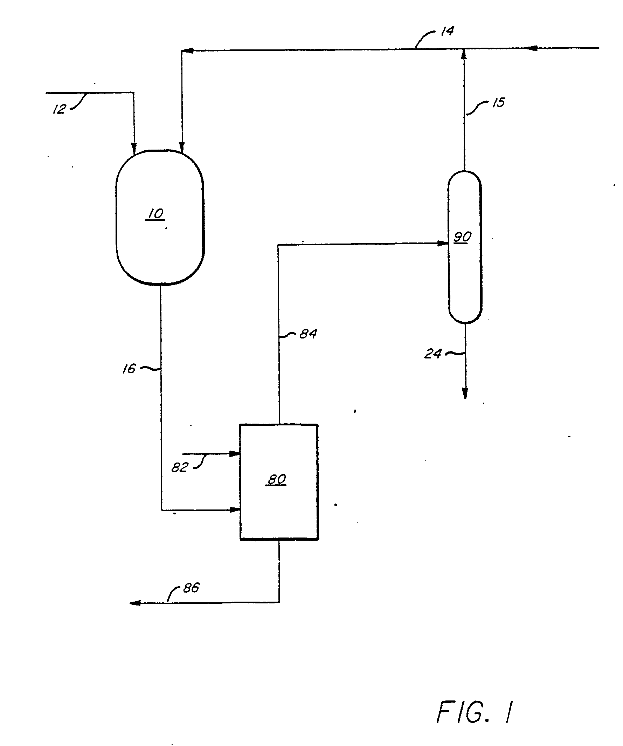

In the drawing, Fig. 1 is a schematic flow sheet with conventional parts omitted showing the general reaction and recovery sequence that is used in the practice of a preferred embodiment of the present invention.

Turning now to Fig. 1, there is shown a schematic flow sheet illustrating a preferred method of practicing the process of the present invention. In the drawing, conventional parts such as valves, pumps, temperature sensors, pressure sensors, heaters, coolers, control and flow regulation apparatus, reboilers, reflux condensers, etc., have been omitted.

In accordance with the present invention, an appropriate reactor 10 is charged with oxygen by way of a charge line 12 and with isobutane by way of a charge line 14 from appropriate sources (not shown) such as a storage tank. Within the reactor 10, and in accordance with known prior art procedures, the oxygen reacts with a portion of the isobutane to provide a reaction mixture which is discharged from the reactor 10 by way of a discharge line 16. The reaction mixture 16 comprises, for example, unreacted isobutane, tertiary butyl alcohol and minor quantities of other reaction components and by-products including acetic acid and formic acid. The liquid reaction product discharged from reactor 10 by line 16 constitutes a crude initial reaction product.

The initial reaction product discharged from the reactor 10 by discharge line 16 will normally have the composition shown in Table I.

TABLE I

| COMPOSITION OF INITIAL REACTION PRODUCT | | |
|---|---|---|
| Component | General Range, Wt.% | Preferred Range, Wt.% |
| Isobutane | 25 - 90 | 50 - 80 |
| t-butyl hydroperoxide | 70 - 0 | 25 - 55 |
| t-butyl alcohol | 0 - 70 | 25 - 55 |
| Others* | 0.5 - 10 | 0.5 - 5 |

* Includes di-tertiary butyl peroxide, acetone, methanol, acetic acid, formic acid and other oxygenated impurities.

The water may suitably be charged through the line 82 at the rate of about 10 to about 200 parts of water per 100 parts of initial reaction product charged by the line 16.

In accordance with the present invention, the initial reaction product 16 is charged to the bottom of a water extraction zone, such as a packed water extraction column 80. Water is charged to the column 80 by way of a water charge line 82 for countercurrent contact with the feed 16. As a consequence of the countercurrent contact, a raffinate phase 84 is formed which is taken overhead and an extract phase 86 is formed, which is composed of water and extracted impurities. The extract is suitably discharged by line 86 for further processing.

By way of example, about 100 kg per hour of a mixture of about 15 wt.% of tertiary butyl alcohol, about 30 wt.% isobutane, about 10 wt.% of tertiary butyl hydroperoxide contaminated with from about 0.05 to about 2 wt.% of carboxylic acid impurities may be charged to the water extraction tower 80 by line 16 and about 50 kg per hour of water may be charged to the water extraction tower 80 by line 82. The water-lean raffinate 84 from the water extraction tower 80, in this instance may comprise, for example, substantially all of the isobutane, tertiary butyl alcohol and tertiary butyl hydroperoxide charged by line 16 and will contain only residual quantities (less than about 0.01 wt.% of carboxylic acid impurities. The water-rich extract solution 86 discharged from water extraction zone 80 by line 86, in this instance, may comprise about 90 to about 95 wt.% of a water and about 1 to about 5 wt.% of tertiary butyl alcohol and about 0.05 to about 2 wt.% of carboxylic acid impurities.

The raffinate phase 84 from water extraction zone 80 is suitably fed to a distillation zone 90 where it is separated into an overhead distillate fraction 15 comprising isobutane which, if desired, may be recycled to isobutane charge line 14.

EP 0 404 417 A1

The higher boiling fraction 24 discharged from the zone 90 will be composed primarily of tertiary butyl hydroperoxide in solution in tertiary butyl alcohol.

If the only primary product to be manufactured is tertiary butyl alcohol, the reaction conditions used in the reactor 10 will be selected so that the tertiary butyl hydroperoxide is converted directly to tertiary butyl alcohol, as disclosed for example, in Worrell U. S. Patent No. 4,296,263 or, in the alternative, the decomposition of the tertiary butyl hydroperoxide is accomplished in a separate digestion zone (not shown), as described for example in Grane et al. U. S. Patent No. 4,294,999 or Grane et al. U. S. Patent No. 4,296,262.

EXAMPLES

The invention will be further illustrated by the following examples, which are given by way of illustration and not as limitations on the scope of this invention.

The following laboratory examples were conducted under ambient conditions of temperature and pressure in a laboratory. Since isobutane has a boiling point of about -11.7° C., it will vaporize rapidly in an open vessel, such as a beaker, even when dissolved in a solvent such as tertiary butyl alcohol. Therefore, in the following experiments, isooctane, a related compound was used rather than isobutane, and was used to simulate the presence of isobutane in the pseudo reaction mixtures.

Example 1

A pseudo reaction mixture was made up of approximately 70% isooctane, approximately 29% tert-butyl alcohol, and approximately 1% acetic acid. 100 ml of this solution was placed in a small separatory funnel and extracted three times with 5.0 ml of demineralized (DM) water. The results are shown in Table II.

TABLE II

| Extracted 3x 5.0 ml $H_2O$ | ml After Extraction | Acid No. (mg/g) |
|---|---|---|
| Reaction mixture | - | 11.27 |
| Reaction mixture after first extraction | - | 10.05 |
| Reaction mixture after second extraction | - | 8.66 |
| Reaction mixture after third extraction | - | 7.85 |
| Water layer 1st extraction | 4.3 | 18.97 |
| Water layer 2nd extraction | 6.6 | 17.09 |
| Water layer 3rd extraction | 6.8 | 15.05 |

Example 2

The procedure was the same as Example 1. The results are shown in Table III.

4

TABLE III

|  | Isooctane[a] | TBA[a] | $H_2O$[a] | Wt%[b] |
|---|---|---|---|---|
| [Extracted 3x10.0 ml $H_2O$] | (Area%) | (Area%) | (Area%) | Acid |
| Reaction Mixture | 67.20 | 31.79 | ~0 | 1.30 |
| Rxn Mix after 1st extr. | 68.80 | 30.05 | 1.09 | 0.89 |
| $H_2O$ layer 1st extr. | ~0 | 18.74 | 81.14 | 1.77 |
| Rxn Mix after 2nd extr. | 71.26 | 26.65 | 2.04 | 0.66 |
| $H_2O$ layer 2nd extr. | ~0 | 17.87 | 82.05 | 1.41 |
| Rxn Mix after 3rd extr. | 74.85 | 23.26 | 1.86 | 0.46 |
| $H_2O$ layer 3rd extr. | ~0 | 16.74 | 83.15 | 1.05 |

a = Obtained from GC
b = Calculated from acid no.
TBA = tertiary butyl alcohol

## Example 3

A pseudo reaction mixture was made up of isooctane, tert-butyl alcohol and formic acid. 100 ml of this solution was placed in a small separating funnel and extracted with 10.0 ml DM water. The results are shown in Table IV.

TABLE IV

|  | Isooctane[a] | TBA[a] | $H_2O$[a] | Wt%[b] |
|---|---|---|---|---|
| [Extracted 3x10.0 ml $H_2O$] | (Area%) | (Area%) | (Area%) | Acid |
| Reaction Mixture | 66.45 | 33.42 | ~0 | 1.30 |
| Rxn Mix after 1st extr. | 67.45 | 30.45 | 2.04 | 1.09 |
| $H_2O$ layer 1st extr. | ~0 | 18.42 | 81.21 | 2.85 |
| Rxn Mix after 2nd extr. | 71.45 | 27.41 | 1.103 | 0.56 |
| $H_2O$ layer 2nd extr. | ~0 | 16.56 | 83.18 | 1.88 |
| Rxn Mix after 3rd extr. | 75.46 | 23.99 | 0.53 | 0.35 |
| $H_2O$ layer 3rd extr. | ~0 | 15.28 | 84.61 | 1.13 |

a = Obtained from GC
b = Calculated from acid no.

## Claims

1. A method for the oxidation of isobutane (14) by reaction with oxygen (12) in a reaction zone (10), to form a crude mixture (16) comprising unreacted isobutane, tertiary butyl hydroperoxide, tertiary butyl alcohol and carboxylic acid impurities, including acetic acid and formic acid, characterized in that said crude mixture (16) and water (82) are contacted in zone (80) and an extract fraction (86) comprising water, tertiary butyl alcohol and said carboxylic acid impurities and a raffinate (84) comprising isobutane, tertiary butyl hydroperoxide and tertiary butyl alcohol, are removed from the water extraction zone (80).

2. A method according to claim 1 characterized in that the water extraction zone (80) is a continuous countercurrent water extraction zone and water (82) is continuously charged to said countercurrent extraction zone at the rate of 10 to 200 parts of water per hour per 100 parts per hour of crude mixture (16).

3. A method according to claim 1 or 2 characterized in that the crude mixture (16) contains from 50 to 80 wt.% of isobutane, from 25 to 55 wt.% of tertiary butyl hydroperoxide, from 25 to 55 wt.% of tertiary butyl alcohol and 0.05 to 2 wt.% of said carboxylic acid impurities, the raffinate (84) contains from 0.01 to 0.05 wt.% of said carboxylic acid impurities and the extract (86) contains 0.5 to 2 wt.% of said carboxylic acid impurities.

4. A method according to any one of claims 1 to 3 characterized in that the raffinate (84) is separated in a distillation zone (90) into an unreacted isobutane distillate fraction (15) and a tertiary butyl hydroperoxide/tertiary butyl alcohol fraction (24).

5. A method according to claim 4 characterized in that the distillate fraction (15) is combined with the isobutane (14) and recycled to a reaction zone (10).

*FIG. 1*

| Category | Citation of document with indication, where appropriate, of relevant passages | Relevant to claim | CLASSIFICATION OF THE APPLICATION (Int Cl.) |
|---|---|---|---|
| | **DOCUMENTS CONSIDERED TO BE RELEVANT** | | EP 90306348.5 |
| P,A | EP - A2 - 0 328 258 (ARCO CHEMICAL TECHNOLOGY) * Claim 1 * | 1 | C 07 C 29/74 C 07 C 31/12 |
| D,A | US - A - 3 449 217 (ROBERT J. HARVEY) * Abstract * | 1,4 | |
| D,A | US - A - 4 296 263 (G. RICHARD WORRELL) * Claim 1 * | 1 | |
| D,A | US - A - 4 296 262 (HENRY R. GRANE et al.) * Claim 1 * | 1 | |
| D,A | US - A - 4 294 999 (HENRY R. GRANE et al.) * Claim 1 * | 1 | |
| P,A | EP - A1 - 0 336 480 (METALLGESELLSCHAFT) * Abstract * | 1,4 | TECHNICAL FIELDS SEARCHED (Int Cl.) |
| D,A | US - A - 3 474 151 (HENRY R. GRANE) * Claim 1 * | 1 | C 07 C 29/00 C 07 C 27/00 C 07 C 31/00 |

The present search report has been drawn up for all claims

| Place of search | Date of completion of the search | Examiner |
|---|---|---|
| VIENNA | 26-09-1990 | REIF |